# EUROPÄISCHE PATENTSCHRIFT

(11) **EP 1 244 606 B1**
(45) Veröffentlichungstag und Bekanntmachung des Hinweises auf die Patenterteilung: **27.04.2005**
(21) Anmeldenummer: 00990810.4
(22) Anmeldetag: 21.12.2000
(51) Int. Cl.: C04B 41/90, A61L 27/32

(54) **HYDROXYLAPATITBESCHICHTUNG VON ALUMINIUMOXID-KERAMIKEN**
COATING ALUMINIUM OXIDE CERAMICS WITH HYDROXYL APATITE
ENDUCTION DE CERAMIQUES D'ALUMINE AVEC DE L'HYDROXYAPATITE

(30) Priorität: 21.12.1999 DE 19961917
(43) Veröffentlichungstag der Anmeldung: 02.10.2002
(73) Patentinhaber: CeramTec AG Innovative Ceramic Engineering, 73207 Plochingen (DE)
(72) Erfinder: ANDERSCH, Hans, 73092 Heiningen (DE); BURGER, Wolfgang, 73207 Plochingen (DE); RICHTER, Herbert, 73257 Köngen (DE); RICHTER, Gert, 75196 Remchingen (DE)
(74) Vertreter: Uppena, Franz, Dr.
(86) Internationale Anmeldenummer: PCT/EP2000/013109
(87) Internationale Veröffentlichungsnummer: WO 2001/046086

(56) Entgegenhaltungen:
- DE-A- 4 342 468
- PATENT ABSTRACTS OF JAPAN vol. 017, no. 011 (C-1015), 8. Januar 1993 (1993-01-08) & JP 04 242659 A (KYOCERA CORP), 31. August 1992 (1992-08-31) -& DATABASE WPI Section Ch, Week 199241 Derwent Publications Ltd., London, GB; Class D21, AN 1992-337148 XP002165622

## Beschreibung

Gegenstand der vorliegenden Erfindung ist ein Verfahren zur Herstellung hydroxylapatitbeschichteter keramischer Bauteile, sowie die nach diesem Verfahren herstellbaren keramischen Bauteile selbst.

Es ist bekannt, dass Prothesen, die eine Hydroxylapatitbeschichtung aufweisen, ein besonders gutes Einwachsverhalten zeigen. Dabei ist aber zu beachten, dass die Hydroxylapatitbeschichtung auf der Prothese fest haftet. Bei der Beschichtung von Ti-Schäften mit Hydroxylapatit ist eine besonders hohe Haftfestigkeit zu erzielen, wenn die Metalloberfläche auf eine Rauhigkeit von Rₐ ≈ 40 - 50 µm eingestellt wird.

Die Haftfestigkeit des Hydroxylapatits auf keramischen Oberflächen, insbesondere auf Al₂O₃-Keramiken ist für den gewünschten Einsatz nicht ausreichend. So ist eine direkte Beschichtung einer Aluminiumoxidkeramik mit Hydroxylapatit, wie sie beispielsweise für die direkte Fixierung des Femurteils einer Knieprothese sehr vorteilhaft wäre, nicht möglich. Auch wenn die Oberflächenrauhigkeit analog zur Rauhigkeit der Ti-Schäfte gestaltet wird, ist die Haftfestigkeit von Hydroxylapatit nicht gewährleistet. Dies konnte bei Versuchen, in denen geschliffene und sandgestrahlte Proben verwendet wurden, nachgewiesen werden. Gegenüber den Titan-Werkstoffen ist die Oberflächenrauhigkeit der so behandelten keramischen Trägerwerkstoffe wesentlich geringer. Beschichtungsversuche mit den Standardparametem für Ti-Schäfte führten zu keiner Haftfestigkeit zwischen Hydroxylapatit und Aluminiumoxidkeramik. Nachdem unter Standardbedingungen keine Schicht auf den Aluminiumoxidträgerkörpern haftete, wurden auch die Spritzparameter in der Plasmaanlage modifiziert. Doch auch die geänderten Verfahrensparameter führten nicht zum Erfolg. Als Ursache für die mangelnde Haftfestigkeit wurde die unterschiedliche Rauhigkeit von Metall und Keramik ermittelt. Mittels konventioneller Schleifverfahren ist eine Oberflächenrauhigkeit mit Rₐ ≈ 30 µm nicht realisierbar.

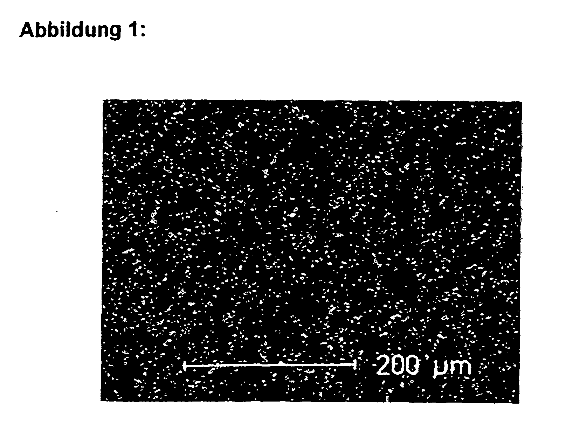

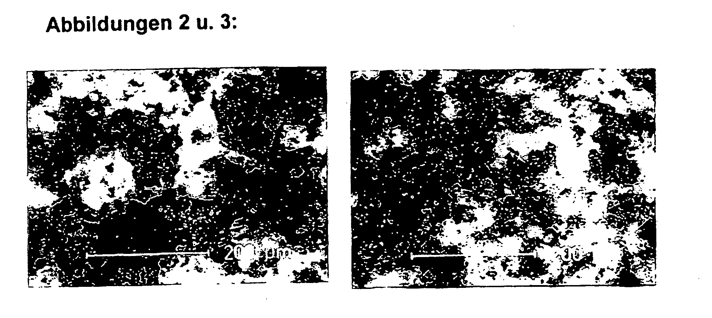

Auch Verfahren, die zu erhöhter Rauhtiefe führen, brachten nicht den gewünschten Erfolg. Für die Erzeugung einer erhöhten definierten Oberflächenrauhigkeit wurden entsprechende Proben zur LASER-Bearbeitung unter verschiedenen Einstellbedingungen hergestellt. Auf diese Weise konnte die Oberfläche der Al₂O₃-Keramik nachhaltig beeinflusst werden. Während sich bei der normalen Schleifbearbeitung lediglich eine Rautiefe von maximal 1 µm erzielen ließ, gelang durch die LASER-Bearbeitung die Erzeugung einer Rauhtiefe von Rₐ ≈ 9 µm. Abbildung 1 zeigt die typische Oberfläche nach der LASER-Behandlung. Die gelaserte Oberfläche der Aluminiumoxidkeramik wurde anschließend der Plasma-Beschichtung mit Hydroxylapatit unterzogen. Erstmalig konnten auf dieser Oberfläche zwar einzelne Stellen detektiert werden, auf denen sich die Hydroxylapatitschicht nachweisen ließ. Allerdings war es auch nach dieser Vorbehandlung nicht möglich, eine durchgehende Schicht aufzubringen. Die Abbildungen 2 und 3 zeigen die Oberflächen der gelaserten und Hydroxylapatit (HA)-beschichteten Proben.

Auch wenn erstmals die Abscheidung von Hydroxylapatit auf den angerauhten Oberflächen der Aluminiumoxidkeramik nachgewiesen werden konnte, so war die Haftfestigkeit der Schicht doch sehr niedrig. Die Quantifizierung der Haftfestigkeit war ebenso wie eine Querschliffpräparation selbst unter den schonendsten Bedingungen nicht möglich; die Schicht löste sich sofort ab. Wiederum wurde bei der Analyse die Rauhtiefe mit der von metallischen Werkstoffen verglichen. Mit einem Rₐ-Wert von 9 µm konnte zwar die Rauhtiefe der TiAl6V4-Legierung (Rₐ ≈ 40 µm) nicht realisiert werden. Von einer weiteren Aufrauhung der Oberfläche im keramischen Substrat musste Abstand genommen werden, da die Aluminiumoxidkeramik im Unterschied zu metallischen Werkstoffen ein absolutes Sprödbruchveralten zeigt. Wird eine "Vorschädigung" von 40 µm induziert kann bereits diese "Fehlstelle" als bruchauslösend wirken Somit verbietet sich unter den Gesichtspunkten der Bruchmechanik eine weitere Erhöhung der Rauhigkeit.

In der DE 43 42 468 A1 und in der JP 4 242 659 A werden Implantate beschrieben, die aus Keramiken wie Aluminiumoxid, Zirkonoxid oder dergleichen bestehen können, die eine erste Überzugsschicht aus Ti, einer Ti-Legierung oder Oxiden davon und eine zweite Überzugsschicht aus beispielsweise Hydroxylapatit aufweisen können. Die Titanschichten können jeweils durch thermische Spritzverfahren aufgebracht werden.

Aufgabe der vorliegenden Erfindung war es daher, ein Verfahren bereitzustellen, mit dem keramische Bauteile zuverlässig mit einer Hydroxylapatitbeschichtung versehen werden können.

Gelöst wurde die der Erfindung zugrundeliegende Aufgabe durch ein Verfahren mit den Merkmalen des Hauptanspruchs. Vorzugsweise Ausgestaltungen sind in den Unteransprüchen beschrieben.

Überraschenderweise gelang es erfindungsgemäß, ein keramisches Bauteil, vorzugsweise ein Bauteil aus Aluminiumoxidkeramik, mit Hydroxylapatit zu beschichten, wenn die Oberfläche des keramischen Bauteils mit einer Ti-Schicht beschichtet wird. Durch das erfindungsgemäße Verfahren ist es überraschenderweise erstmals möglich, Hydroxylapatit auf der Oberfläche eines keramischen Bauteils mit genügender Haftfestigkeit abzuscheiden.

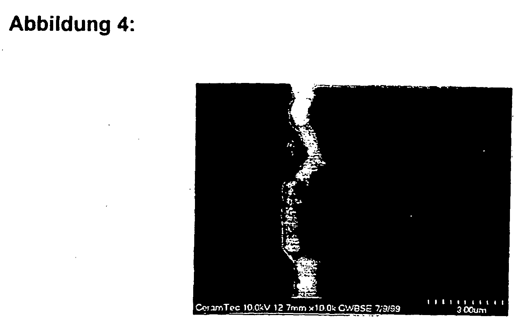

Zunächst wurden erfindungsgemäß keramische Bauteile mit einer dünnen Ti-Schicht, beispielsweise mittels PVD (Physical Vapor Deposition), versehen. Erfindungsgemäß kann dabei die Oberfläche des keramischen Bauteils vorher aufgerauht, beispielsweise geschliffen oder gelasert, werden. Die Dicke der Ti-Schicht lag bei ca. 1 µm; eine 5 µm dicke Schicht führt ebenfalls zum Erfolg. Abbildung 4 zeigt den Querschliff einer derartig beschichteten Probe.

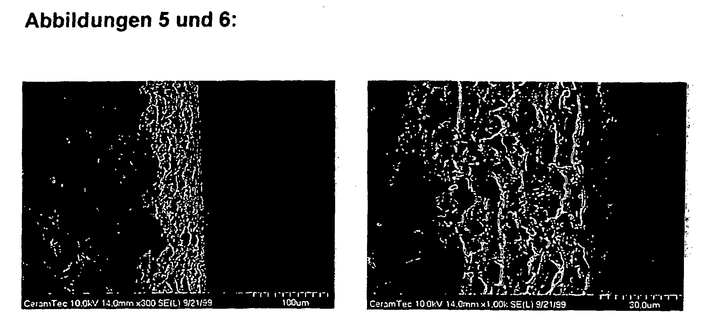

Auf diese Zwischenschicht wurde die Hydroxylapatitschicht aufgespritzt. Der Querschliff dieses Schichtaufbaus ist in den Abbildungen 5 und 6 bei unterschiedlichen Vergrößerungen dargestellt.

Vorzugsweise wird die Ti-Zwischenschicht vor der Beschichtung mit Hydroxylapatit, beispielsweise durch eine Plasma-Beschichtung, noch einem Sandstrahlprozeß unterzogen, um die Haftfestigkeit weiter zu steigern. Eine besonders hohe Haftfestigkeit wird erzielt, wenn die Ti-Schicht auf eine Rauhigkeit von Rₐ ≈ 40 - 50 µm eingestellt wird.

Ein Kratztest auf der Hydroxylapatitschicht bestätigte die vorzügliche Haftfestigkeit der Schicht. Eine Querschliffpräparation war ohne Probleme möglich. Die Messung der Haftfestigkeit wurde an fünf verschiedenen Proben bestimmt. Die Einzelwerte sind in Tabelle 1 zusammengefasst.

**Tabelle 1:**

| Haftfestigkeit von Hydroxylapatit auf Al₂O₃ mit Ti-Zwischenschicht | | |
|---|---|---|
| **Probe** | **Kraft [N]** | **Spannung [MPa]** |
| 1 | 718 | 2,3 |
| 2 | 1203 | 3,8 |
| 3 | 932 | 3 |
| 4 | 1490 | 4,7 |
| 5 | 390 | 1,2 |

Aus den Einzelwerten der Haftfestigkeitsmessungen ist zu entnehmen, dass überraschenderweise Spannungen realisiert werden können, die im Bereich der Haftfestigkeit von Hydroxylapatitschichten auf TiAl6V4-Legierungen liegen.

Erfindungsgemäß kann anstelle der konventionellen Ti-Zwischenschicht auch eine Zwischenschicht aus der TiAl6V4-Legierung, beispielsweise im PVD-Verfahren, abgeschieden werden.

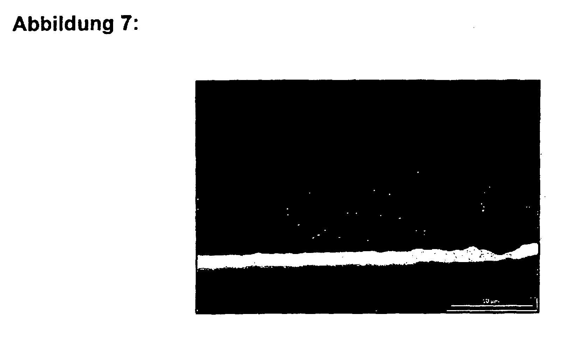

Abbildung 7 zeigt den typischen Schichtaufbau in der Querschliffpräparation. Die dazugehörigen Haftfestigkeiten sind in Tabelle 2 zusammengestellt.

**Tabelle 2:**

| Haftfestigkeit von Hydroxylapatit auf Al₂O₃ mit TiAl6V4-Zwischenschicht | | |
|---|---|---|
| **Probe** | **Kraft [N]** | **Spannung [MPa]** |
| 1 | 582 | 1,9 |
| 2 | 700 | 2,2 |
| 3 | 400 | 1,3 |
| 4 | 498 | 1,6 |

Für die Versuche wurde ein keramisches Bauteil in Form eines zylindrischen Probenkörpers verwendet. Die Zylinder mit einem Durchmesser von 20 mm und einer Dicke von 2 mm wurden über die konventionelle Preß-Drehfertigung als Grünkörper hergestellt. Anschließend wurden die Grünkörper vorgesintert, heißisostatisch nachverdichtet und geglüht. Die Sinterrohlinge wurden dann mit Diamantwerkzeugen zur Erreichung der endgültigen Geometrie bearbeitet. Auch andere Verfahren zur Herstellung keramischer Bauteile können selbstverständlich eingesetzt werden. Als Werkstoff wurde ein an sich bekannter Aluminiumoxid-Werkstoff eingesetzt, wie er beispielsweise als Biolox®-Werkstoff bekannt ist.

Mit der vorliegenden Erfindung ist es damit erstmals möglich, über das Einbringen einer Ti-Zwischenschicht Hydroxylapatit direkt auf keramische Bauteile abzuscheiden. Die nach dem erfindunsgemäßen Verfahren herstellbaren keramischen Bauteile sind ebenfalls Gegenstand der vorliegenden Erfindung.

Damit können erfindungsgemäß erstmals keramische Bauteile bereitgestellt werden, die für medizinische Zwecke, beispielsweise als Prothesen, eingesetzt werden können. Solche Prothesen zeigen ein verbessertes Einwachsverhalten.

## Patentansprüche

1. Verfahren zur Herstellung hydroxylapatitbeschichteter keramischer Bauteile, **dadurch gekennzeichnet, dass** in einem ersten Schritt das keramische Bauteil zunächst mit einer Ti-Schicht oder einer Schicht aus einer TiAl6V4-Legierung versehen wird, die Ti-Schicht oder die Schicht aus einer TiAl6V4-Legierung auf eine Rauhigkeit von Rₐ 40 bis 50 µm eingestellt wird und in einem zweiten Schritt auf der Ti-Schicht oder der Schicht aus der TiAl6V4-Legierung das Hydroxylapatit aufgebracht wird.

2. Verfahren gemäß Anspruch 1, **dadurch gekennzeichnet, dass** die Ti-Schicht oder die Schicht aus der TiAl6V4-Legierung mit Hilfe des PVD (Physical Vapor Deposition)-Verfahrens aufgebracht wird.

3. Verfahren gemäß Anspruch 1 oder 2, **dadurch gekennzeichnet, dass** die im ersten Schritt aufgebrachte Ti- bzw. TiAl6V4-Schicht bis 5 µm dick ist.

4. Verfahren gemäß einem oder mehreren der Ansprüche 1 bis 3, **dadurch gekennzeichnet, dass** die im ersten Schritt aufgebrachte Ti- bzw. TiAl6V4-Schicht zwischen 1 und 5 µm dick ist.

5. Verfahren gemäß einem oder mehreren der Ansprüche 1 bis 4, **dadurch gekennzeichnet, dass** das Hydroxylapatit auf die im ersten Schritt aufgebrachte Ti- bzw. TiAl6V4-Schicht aufgespritzt wird.

6. Verfahren gemäß einem oder mehreren der Ansprüche 1 bis 5, **dadurch gekennzeichnet, dass** das Hydroxylapatit auf die im ersten Schritt aufgebrachte Ti- bzw. TiAl6V4-Schicht mit Hilfe der Plasma-Beschichtung aufgebracht wird.

7. Verfahren gemäß einem oder mehreren der Ansprüche 1 bis 6, **dadurch gekennzeichnet, dass** das keramische Bauteil eine Aluminiumoxid-Keramik ist.

8. Verfahren gemäß einem oder mehreren der Ansprüche 1 bis 7, **dadurch gekennzeichnet, dass** das keramische Bauteil ein medizinisches Bauteil ist.

9. Verfahren gemäß einem oder mehreren der Ansprüche 1 bis 8, **dadurch gekennzeichnet, dass** das keramische Bauteil eine Prothese ist.

10. Hydroxylapatitbeschichtetes keramisches Bauteil, herstellbar nach einem oder mehreren der vorhergehenden Ansprüche.

## Claims

1. Method for producing hydroxylapatite-coated ceramic components, **characterised in that** in a first step the ceramic component is first provided with a Ti-layer or a layer consisting of a TiAl6V4-alloy, the Ti-layer or the layer consisting of a TiAl6V4-alloy is adjusted to a roughness of Rₐ 40 to 50 µm, and in a second step the hydroxylapatite is applied to the Ti-layer or the layer consisting of the TiAl6V4-alloy.

2. Method according to claim 1, **characterised in that** the Ti-layer or the layer consisting of the TiAl6V4-alloy is applied by means of the PVD (Physical.Vapour Deposition) process.

3. Method according to claim 1 or 2, **characterised in that** the Ti- or TiAl6V4-layer applied in the first step is up to 5 µm thick.

4. Method according to one or more of claims 1 to 3, **characterised in that** the Ti- or TiAl6V4-layer applied in the first step is between 1 and 5 µm thick.

5. Method according to one or more of claims 1 to 4, **characterised in that** the hydroxylapatite is sprayed onto the Ti- or TiAl6V4-layer applied in the first step.

6. Method according to one or more of claims 1 to 5, **characterised in that** the hydroxylapatite is applied by means of plasma-coating to the Ti- or TiAl6V4-layer applied in the first step.

7. Method according to one or more of claims 1 to 6, **characterised in that** the ceramic component is an aluminium-oxide ceramic material.

8. Method according to one or more of claims 1 to 7, **characterised in that** the ceramic component is a medical component.

9. Method according to one or more of claims 1 to 8, **characterised in that** the ceramic component is a prosthesis.

10. Hydroxylapatite-coated ceramic component, producible according to one or more of the preceding claims.

## Revendications

1. Procédé de fabrication d'une pièce de structure en céramique revêtue d'hydroxyapatite, **caractérisé en ce que**, dans une première étape, on enrobe d'abord la pièce de structure d'une couche de titane ou d'une couche d'alliage TiAl6V4, couche de titane ou d'alliage TiAl6V4 dont on ajuste la rugosité à une valeur Rₐ de 40 à 50 µm, et dans une deuxième étape, on dépose de l'hydroxyapatite sur cette couche de titane ou d'alliage TiAl6V4.

2. Procédé conforme à la revendication 1, **caractérisé en ce qu'**on a recours à un procédé PVD (dépôt physique à partir d'une phase vapeur) pour déposer la couche de titane ou la couche d'alliage TiAl6V4.

3. Procédé conforme à la revendication 1 ou 2, **caractérisé en ce que** l'épaisseur de la couche de titane ou d'alliage TiAl6V4 déposée lors de la première étape vaut jusqu'à 5 µm.

4. Procédé conforme à l'une ou plusieurs des revendications 1 à 3, **caractérisé en ce que** l'épaisseur de la couche de titane ou d'alliage TiAl6V4 déposée lors de la première étape vaut de 1 à 5 µm.

5. Procédé conforme à l'une ou plusieurs des revendications 1 à 4, **caractérisé en ce que** l'on dépose de l'hydroxyapatite par projection sur la couche de titane ou d'alliage TiAl6V4 déposée lors de la première étape.

6. Procédé conforme à l'une ou plusieurs des revendications 1 à 5, **caractérisé en ce que**, pour déposer de l'hydroxyapatite sur la couche de titane ou d'alliage TiAl6V4 déposée lors de la première étape, on a recours à un procédé de revêtement par plasma.

7. Procédé conforme à l'une ou plusieurs des revendications 1 à 6, **caractérisé en ce que** la céramique de la pièce de structure est une céramique d'alumine.

8. Procédé conforme à l'une ou plusieurs des revendications 1 à 7, **caractérisé en ce que** la pièce de structure en céramique est une pièce de structure à usage médical.

9. Procédé conforme à l'une ou plusieurs des revendications 1 à 8, **caractérisé en ce que** la pièce de structure en céramique est une prothèse.

10. Pièce de structure en céramique revêtue d'hydroxyapatite, que l'on peut fabriquer selon un procédé conforme à l'une ou plusieurs des revendications précédentes.
